(19) Europäisches Patentamt
European Patent Office
Office européen des brevets

(11) Veröffentlichungsnummer: **0 622 375 A1**

## (12) EUROPÄISCHE PATENTANMELDUNG

(21) Anmeldenummer: **94102320.2**

(22) Anmeldetag: **16.02.94**

(51) Int. Cl.5: **C07K 7/08**, A61K 37/02, C12P 21/02, //(C12P21/02, C12R1:645)

(30) Priorität: **20.02.93 DE 4305352**

(43) Veröffentlichungstag der Anmeldung: **02.11.94 Patentblatt 94/44**

(84) Benannte Vertragsstaaten: **AT BE CH DE DK ES FR GB GR IE IT LI LU MC NL PT SE**

(71) Anmelder: **HOECHST AKTIENGESELLSCHAFT Brüningstrasse 50 D-65929 Frankfurt am Main (DE)**

(72) Erfinder: **Metzger, Jörg Justinus-Kerner-Strasse 31 D-72070 Tübingen (DE)**
Erfinder: **Schlegel, Brigitte, Dr. Werner-Seelenbinder-Strasse 20, D-07747 Jena (DE)**
Erfinder: **Fleck, Werner Friedrich, Dr. Löberwallgraben 10 D-99096 Erfurt (DE)**
Erfinder: **Dornberger, Klaus Jürgen, Dr. Diesterweg 4 D-07743 Jena (DE)**
Erfinder: **Ihn, Wolfgang, Dr. Heinrich-von-Eggeling-Strasse 7 D-07749 Jena (DE)**
Erfinder: **Schade, Wolfgang, Dr. Naumburger Strasse 27 D-07745 Jena (DE)**
Erfinder: **Gräfe, Udo, Prof.Dr. Hermann-Löns-Strasse 43b D-07745 Jena (DE)**

(54) **Chrysospermine, Peptidwirkstoffe aus Apiocrea chrysosperma mit pharmakologischer Wirkung, ein Verfahren zu Herstellung und Verwendung derselben.**

(57) Die Erfindung betrifft die Peptidwirkstoffe Chrysospermine der Formel I

AcPHe-Aib-Ser-Aib-x-Leu-Gln-Gly-Aib-Aib-Ala-Ala-Aib-Pro-y-Aib-Aib-Gln-Trp-ol

in der x und y unabhängig voneinander Aib oder Iva bedeuten, welche von dem Pilz Apiocrea chrysosperma während der Fermentation synthetisiert werden und sich in der Kulturbrühe anhäufen, ein Herstellungsverfahren, die Verwendung der Chrysospermine als pharmakologische Wirkstoffe, insbesondere als Wirkstoff gegen Bakterien, Pilze und/oder Nematoden sowie zur Behandlung von Abstoßreaktionen bei Organverpflanzungen und von Tumorerkrankungen und den Mikroorganismus Apiocrea chrysosperma DSM 7444.

EP 0 622 375 A1

Die Erfindung betrifft die Peptidwirkstoffe Chrysospermine, welche von dem Pilz Apiocrea chrysosperma während der Fermentation synthetisiert werden und sich in der Kulturbrühe anhäufen, ein Herstellungsverfahren, die Verwendung der Chrysospermine als pharmakologische Wirkstoffe, insbesondere als Wirkstoff gegen Bakterien, Pilze und/oder Nematoden sowie zur Behandlung von Abstoßungsreaktionen bei Organverpflanzungen und von Tumorerkrankungen und den Mikroorganismus Apiocrea chrysosperma DSM 7444.

Peptide mit bis zu 20, zum Teil strukturell ungewöhnlichen Aminosäuren werden von Bakterien und Pilzen über deren Sekundärwirkstoffwechsel mittels nichtribosomaler Peptid-Synthetasen produziert. Viele der bisher bekannten Sekundärmetabolite mit Peptidstruktur besitzen interessante biologische Wirkungen als Antibiotika, Enzyminhibitoren, Kardiotonika, Immunmodulatoren, Insektizide, Nematozide u.a.m. (siehe z.B. Gräfe, U. Biochemie der Antibiotika, Spektrum Heidelberg, 1992; Sasaki et al., J. Antibiot., 45, 692 bis 697, 1992; Hamano et al., J. Antibiot., 45, 899 bis 905, 1992; Tomoda et al., J.Antibiot., 45, 1207 bis 1215, 1992.

Pharmakologisch aktive Sekundärmetabolite lassen sich mit Hilfe biologischer Screeningverfahren nachweisen, beispielsweise kann in Stämmen der Gattung Phoma die Bildung von Pigmenten stimuliert werden. (Hübner, R.; Schlegel, B.; Fleck, W. F.: Fungal cells as screening models for microbial metabolites affecting the differentiation; S. 90 in : Abstracts of 2nd International Bioactive Metabolites from Microorganisms, May 2 bis 7, 1988 Gera, FRG).

Innerhalb der Strukturklasse der Peptidwirkstoffe sind die sogenannten Peptaibole (Peptaibophole bei Anwesenheit von Phenylalaninol als C-terminaler Substituent) dadurch ausgezeichnet, daß sie ungewöhnlich viele Aminosäuren (bis zu 20, darunter einen hohen Anteil an alpha-Aminoisobuttersäure) enthalten (Brückner, H., König, W.A., Greiner, M., Jung, G. Angew. Chem. Int. Ed. Engl. 18 (1979), 476 bis 477).

Für einige Vertreter der Paptaibophole vom Alamethicin/Suzukacillin-Typ (Alamethicine, Suzukacillin, Paracelsine, Hypelcin, Trichotoxin, Antiamoaebin, Emerimycin, Cerexine, Zervamicin und Trichorzianine) wurden antibakterielle, antifungische, antiprotozoale und nematozide Aktivitäten sowie antitumorale, antiinflammatorische, hämolytische und antihelmintische Wirkungen und die Bildung helikal organisierter Ionenkanäle in biologischen Membranen nachgewiesen. (Gale, E.F. et al. in: The Molecular Basis of Antibiotic Action; J. Wiley, New York, Sydney, Toronto; 1981), Bycroft, B.W.: Dictionary of Antibiotics and Related Substances; Chapman and Hall, London, New York; 1988).

Die bisher bekannten Peptidwirksoffe weisen allerdings oft Nachteile auf, die sich in unbefriedigender Wirkhöhe, hoher Toxizität und/oder unerwünschten Nebenwirkungen äußern.

Für Peptidwirkstoffe mit bis zu 20, teilweise ungewöhnlichen Aminosäuren wurden noch kaum Wirkungen als Antitumormittel beschrieben. Viele der bekannten Antitumormittel erzeugen während der Therapie als Nebenwirkungen Übelkeit, Erbrechen und Durchfall, die auch eine ärztliche Behandlung im Krankenhaus nötig machen. Ferner verändern diese Arzneimittel auch die Wachstumsgeschwindigkeit von anderen körpereigenen Zellen, welche dann zu Symptomen wie beispielsweise Haarausfall oder Blutarmut (Anämie) führen.

Es ist bisher auch nicht bekannt, diese Peptide als Wirkstoff bzw. Arzneimittel zur Behandlung von Abstoßungsreaktionen einzusetzen. In den USA wurden 1990 15 000 Organverpflanzungen vorgenommen. Die Mehrzahl der Verpflanzungen betrifft die Niere, aber es werden auch zunehmend Herz, Haut, Lunge, Leber und Bauchspeicheldrüse verpflanzt. Bei einer Vielzahl der Patienten kommt es dabei zu einer Abstoßungsreaktion des Körpers gegen das übertragene Organ, das von einem anderen Menschen stammt. Man unterscheidet dabei unter drei Formen der Abstoßungsreaktion: hyperakute, akute und chronische Abstoßung.

Die hyperaktute Abstoßung wird im wesentlichen durch im Blut zirkulierende Antikörper verursacht, die gegen das Gewebe des übertragenen Organs (Transplanat) gerichtet sind, und in sehr kurzer Zeit - häufig in Minuten - zu Nekrosen am Transplantat führen.

Aufgabe der Erfindung ist es, nach neuen mikrobiellen Peptidwirkstoffen mit verbesserten Eigenschaften und neuen Wirkmechanismen zu suchen.

Diese Aufgabe wird erfindungsgemäß gelöst, indem man Apiocrea chrysoperma in einer Nährlösung mit Kohlenstoff- und Stickstoffquelle sowie den üblichen anorganischen Salzen fermentiert, bis sich die Peptidwirkstoffe Chrysospermine in der Kultur anhäufen, anschließend die Chrysospermine aus der Kulturbrühe isoliert und gegebenenfalls die Chrysospermine in die Chrysospermine A, B, C und D auftrennt. Die Peptidwirkstoffe besitzen pharmakologische Wirksamkeit und können insbesondere als Wirkstoff gegen Bakterien, Pilze und/oder Nematoden sowie zur Behandlung von Abstoßungsreaktionen bei Organverpflanzungen und von Tumorerkrankungen eingesetzt werden.

Die Erfindung betrifft somit:

1. Eine Verbindung der allgemeinen Formel I

AcPhe-Aib-Ser-Aib-x-Leu-Gln-Gly-Aib-Aib-Ala-Ala-Aib-y-Aib-Aib-Aib-Gln-Trp-ol

in der x und y unabhängig voneinander Aib oder Iva bedeuten.

2. Ein Verfahren zur Herstellung einer Verbindung der allgemeinen Formel I, das dadurch gekennzeichnet ist, daß man Apiocrea chrysosperma in einem Nährmedium kultiviert, bis sich eine Verbindung der allgemeinen Formel I in der Kulturbrühe anhäuft und diese aus der Kulturbrühe isoliert.

3. Eine Verwendung einer Verbindung der allgemeinen Formel I als pharmakologisch wirksamen Stoff.

4. Apiocrea chrysosperma DSM 7444.

Die Erfindung wird im folgenden detailliert beschrieben, insbesondere in ihren bevorzugten Ausführungsformen. Ferner wird sie durch den Inhalt der Patentansprüche bestimmt.

Begriffe und Abkürzungen werden wie folgt definiert:

- Verbindungen der allgemeinen Formel I werden auch als Chrysospermine oder Peptidwirkstoffe bezeichnet.
- Als Kulturbrühe wird das Nährmedium, welches das darin gewachsene Pilzmyzel enthält, bezeichnet.
- AcPhe steht für N-Acetylphenylalanin, Aib für α-Aminobuttersäure, Iva für Isovalin und Trp-ol für Tryptophanol.
- Chrysospermin A (1896 Dalton) bezeichnet eine Verbindung der allgemeinen Formel I, in der an Position x und y Aib steht.
- Chrysospermin B (1910 Dalton) bezeichnet eine Verbindung der allgemeinen Formel I, in der an Position x Aib und Position y Iva steht.
- Chrysospermin C (1910 Dalton) bezeichnet eine Verbindung der allgemeinen Formel I, in der an Position x Iva und Position y Aib steht.
- Chrysospermin D (1924 Dalton) bezeichnet eine Verbindung der allgemeinen Formel I, in der an Position x und y Iva steht.

Unter dem Begriff Organ, werden alle Organe bei Säugetieren, insbesondere des Menschen verstanden, beispielsweise Niere, Herz, Haut, Leber, Bauchspeicheldrüse, Muskel, Knochen, Darm oder Magen, aber auch Blut oder Haare.

Unter Abstoßungsreaktion sind alle Abwehrmaßnahmen des Empfängerorganismusses gemeint, die letztlich zum Zell- oder Gewebsuntergang des übertragenen Organs führen bzw. die Lebensfähigkeit des übertragenen Organs beeinträchtigen.

Chrysospermine werden durch Apiocrea chrysosperma, bevorzugt Apiocrea chrysosperma DSM 7444 produziert.

Apiocrea chrysosperma (ältere Bezeichnung Hypomyces chrysospermus, in: Compendium of Soil Fungi, K.H. Domsch, W. Gams, T.-H. Anderson (Hrsg.) Academic Press, Vol. 1, 398 bis 399) ist als parasitärer Mikroorganismus in den Fruchtkörpern von Boletales in gemäßigten Klimazonen sehr verbreitet und wird aus Erdproben gewonnen.

Morphologisch zeichnet sich die anamorphe Form von Apiocrea chrysosperma aus durch

- verzweigte Konidiophoren,
- gehäuft auftretende, gelblich runde, vereinzelt auch blastische Konidien,
- warzenförmige oder stachlige Konidien mit dicker, mehrschichtiger Wand.

Reinkulturen aus solchen Erdproben lassen sich durch dem Fachmann vertrauten Methoden durch Anlegung von Verdünnungsreihen, Ausplattieren und Bebrüten auf Nähragarmedien isolieren.

Aufgrund von aufeinanderfolgenden Isolierungsschritten kann man von Apiocrea chrysosperma eine Pilzkolonie isolieren, die die Peptidwirkstoffe Chrysospermine sehr effizient in der Kulturbrühe anhäufen.

Die stark produzierende Pilzkolonie wird vermehrt. Ein Isolat von Apiocrea chrysosperma DSM 7444 wurde bei der Deutschen Sammlung von Mikroorganismen und Zellkulturen GmbH, Marschroder Weg 1B, 3300 Braunschweig, Deutschland gemäß den Bedingungen des Budapester Vertrags am 28.01.1993 hinterlegt.

Bei Kultivierung auf Agarnährböden mit Glukose und Malzextrakt als Kohlenstoff-Quelle und Hefeextrakt als Stickstoff-Quelle ist das Mycel des Pilzes nach 14tägiger Bebrütung bei 25°C weiß, später dottergelb, bei Anwesenheit stumpfwarziger Konidien. Die teleomorphe Form wird in der Kultur nicht gebildet.

In einem Nährmedium mit einer Kohlenstoffquelle, einer Stickstoffquelle und den üblichen Mineralsalzen produzieren Apiocrea chrysosperma, bevorzugt Apiocrea chrysosperma DSM 7444, Peptidwirkstoffe der allgemeinen Formel I. Anstelle des Stammes Apiocrea chrysosperma DSM 7444 können natürlich auch

EP 0 622 375 A1

dessen Mutanten und Varianten eingesetzt werden.

Als Mutanten und Varianten gelten all jene Mikroorganismen der Spezies, die in der Lage sind, die Peptidwirkstoffe Chrysospermine zu synthetisieren.

Solche Mutanten können in an sich bekannter Weise durch physikalische Mittel, beispielsweise Bestrahlung, wie mit Ultraviolet- oder Röntgenstrahlen, oder chemischen Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Das Screening nach Mutanten und Varianten, die eine Verbindung der allgemeinen Formel I synthetisieren erfolgt durch Bestimmung der Pigmentbildung in Testorganismen der Gattung Phoma, bevorzugt Phoma sp., besonders bevorzugt Phoma destructiva, die durch die in den Überstand ausgeschiedenen Peptidwirkstoffe der allgemeinen Formel I stimuliert wird.

Alternativ kann die biologische Aktivität der in den Überstand ausgeschiedenen Peptidwirkstoffe auch durch Austesten der antibiotischen Wirkungen auf bekannte Testkeime anhand dem Fachmann vertrauten Methoden überprüft werden.

Als bevorzugte Kohlenstoffquellen für die aerobe Fermentation eignen sich kohlenhydrathaltige Naturprodukte wie Malzextrakt aber auch assimilierbare Kohlenhydrate und Alkohole wie z.B. Glukose, Laktose, Glycerin oder deren Gemische in beliebigen Verhältnis.

Als Stickstoffquelle eignen sich Aminosäuren, Peptide und Proteine sowie deren Abbauprodukte wie Tryptone oder Peptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate.

Die Bildung einer Verbindung der allgemeinen Formel I erfolgt besonders gut in Nährmedien, die 0.2 bis 6 % Malzextrakt, bevorzugt 2 bis 4 % Malzextrakt und 0.01 bis 1 % Hefeextrakt, bevorzugt 0.1 bis 0.4 % Hefeextrakt enthalten.

Die Kultivierung erfolgt aerob, beispielsweise submers unter Schütteln und Rühren in Schüttelkolben, vorzugsweise aber emers als Standkultur. Sie wird in einem Temperaturbereich zwischen 15 bis 35°C, bevorzugt zwischen 23 bis 33°C, besonders bevorzugt zwischen 27 bis 30°C durchgeführt.

Der pH-Wert liegt zwischen 4 und 7, vorzugsweise zwischen 5,5 und 6,5.

Der Mikroorganismus wird unter diesen Bedingungen 5 bis 21 Tage, vorzugsweise 7 bis 18 Tage kultiviert.

Die Fermentation kann im Labormaßstab (Kulturvolumina zwischen 100 ml und 200l) aber auch im Produktionsmaßstab (Volumina bis mehrere m$^3$) durchgeführt werden.

Vorteilhaft kultiviert man in mehreren Stufen, d.h. man stellt zunächst ein oder mehrere Vorkulturen in einem flüssigen Nährmedium her, die dann in das eigentliche Produktionsmedium, die Hauptkultur, beispielsweise im Verhältnis 1:20 überimpft werden.

Die Vorkultur erhält man, indem man ein versportes Mycel von z.B. Malzagar Nährböden in eine Nährlösung überführt, beispielsweise durch Einimpfen von mit Myzel bewachsenen Agarstücken und diese 11 bis 21 Tage, vorzugsweise 15 bis 18 Tage inkubiert.

Das versporte Mycel kann beispielsweise erhalten werden, indem man den Stamm etwa 11 bis 21 Tage, vorzugsweise 15 bis 18 Tage auf einen festen Nährboden, z.B. Malzagar wachsen läßt.

Der Fermentationsverlauf und die Entstehung der Peptidwirkstoffe kann entsprechend dem Fachmann bekannten chromatographischen Methoden, wie z.B. der Dünnschichtchromatographie (DC) oder Hochleistungsflüssigkeitschromatographie (HPLC) oder Austesten der biologischen Aktivität gegenüber Testorganismen verfolgt werden.

Die Peptidwirkstoffe der allgemeinen Formel I sind in der Kulturbrühe, vorzugsweise im Myzel enthalten.

Die Isolierung der Peptidwirkstoffe aus dem Kulturmedium erfolgt nach bekannten Methoden unter Berücksichtigung der chemischen, physikalischen und biologischen Eigenschaften der Produkte.

Zum Testen der Wirkstoffkonzentration im Kulturmedium oder in den einzelnen Isolierungsstufen kann die Dünnschichtchromatographie, beispielsweise an Kieselgel mit Chloroform/Methanol-Mischungen als Laufmittel verwendet werden. Die Detektion bei der dünnschichtchromatographischen Auftrennung kann beispielsweise durch Färbereagentien, wie 2 % Vanillin/konz. Schwefelsäure erfolgen.

Zur Isolierung der Peptidwirkstoffe werden Kulturbrühe und Mycel am Ende der Fermentation ein- bis fünfmal, vorzugsweise dreimal mit organischen lipophilen Lösungsmitteln wie Ethyl- und Butylacetat oder Dichlormethan, vorzugsweise Ethylacetat extrahiert.

Nach Einengung des Extraktes und Ausfällung der Chrysopermine A, B, C und D mit aliphatischen Kohlenwasserstoffen wie n-Hexan oder n-Heptan erfolgt die Auftrennung der Chrysospermine auf chromatographischem Wege unter Einsatz üblicher chromatographischer Adsorbienten bzw. Trägermaterialien wie

4

z.B. Kieselgele oder organophile Dextrangele.

Durch sequentielle Anwendung der Säulenchromatographie an Kieselgel oder Gelchromatographie an organophilen Dextrangelen wird ein Gemisch der Chrysospermine A, B, C und D (Molmassen 1896, 1910, 1910 bzw. 1924 Dalton) erhalten. Eine Auftrennung in die einzelnen Komponente erfolgt durch präparative Hochleistungsflüssigkeitschromatographie (HPLC).

Die chemische Identität der durch Apiocrea chrysosperma, bevorzugt Apiocrea chrysosperma DSM 7444 gebildeten Peptidwirkstoffe Chrysospermin A, B, C und D wird durch chemischen Abbau und Analyse der gebildeten Aminosäuren, Massenspektrometrie (FAB-MS, Electrospray-MS, MS-MS) und NMR-Untersuchungen zweifelsfrei bestätigt.

Die antibakterielle bzw. antifungale Wirkung kann durch dem Fachmann bekannte Methoden, wie beispielsweise Hemmhoftests oder Plattendiffusionstests in vitro gezeigt werden.

Eine Verbindung der allgemeinen Formel I ist gegen grampositive Bakterien, z.B. Bacillus subtilis ATCC 6633, Staphylococcus aureus sowie Hefen, z.B. Klyveromyces marxianus oder filamentösen Pilzen, z.B. Glomerella cingulata, als auch gegen gramnegative Bakterien, z.B. Klebsiella pneumoniae wirksam.

Die minimale Hemmkonzentration liegt im Bereich von > 0,05 bis < 20 $\mu$g/Stanzloch.

Eine nematozide Wirkung kann ebenfalls durch einen in vitro Test, in dem die Vermehrung und Beweglichkeit der Nematoden bestimmt wird, gezeigt werden. Die Peptidwirkstoffe Chrysospermine sind bei Mengen (> 10 $\mu$g/ml) gegen Nematoden, z.B. Caenorhabditis wirksam.

Als weiteres Charakteristikum induzieren Chrysospermine in Konzentrationen > 10 $\mu$g/Stanzloch im Agarplatten-Diffusionstest die Bildung brauner Pigmente in Phoma sp.

Die Chrysospermine zeigen außerdem eine Inhibierung der Bildung allophiler oder xenophiler Antikörper. Damit ergibt sich die Möglichkeit, die hyperakute, aktue und chronische Abstoßungsreaktion des Empfängers gegen das verpflanzte Organ wirksam zu behandeln.

Die Verbindung der Formel I deren physiologisch verträgliche Salze eignen sich in besonderer Weise zur Behandlung von einer Vielzahl von Krebserkrankungen. Zu den Krebsarten, die besonders durch diese Verbindungen gehemmt werden, gehören beispielsweise Leukämie, insbesondere chronische Leukämie des T-und B-Zelltyps, Lymphknotenkrebs, z.B. Hodgkin's oder non-Hodgkin's Lymphom, Karzinome, Sarkome oder Hautkrebs. Die Wirkstoffe können entweder für sich alleine, beispielsweise in Form von Mikrokapseln, in Mischungen miteinander oder in Kombination mit geeigneten Hilfs- und/oder Trägerstoffen verabreicht werden.

Diese aus dem Stand der Technik bekannten Symptome können bei der Behandlung von Menschen und Tieren mit einer Verbindung der Formel I nicht beobachtet werden. Diese Wirkstoffe haben im Gegensatz zu den bisher bekannten cytotoxischen Antikrebsmitteln nicht die Eigenschaft, das Immunsystem zu beeinträchtigen (Bartlett, Int. J. Immunopharmac., 1986, 8: 199 bis 204). Damit eröffnen sich neue Wege der Tumortherapie, denn das körpereigene Abwehrsystem wird nicht beeinträchtigt, während Tumorzellen am Wachstum gehindert werden.

Als Wirksamkeitstest von Chemotherapeutika wird der in vitro Proliferationstest von Zellkulturen herangezogen.

Chrysospermine eignen sich aufgrund ihrer wertvollen pharmakologischen Eigenschaften sehr gut zur Anwendung als Arzneimittel.

Eine erfindungsgemäße Verbindung oder deren Salze kann grundsätzlich als solche in Substanz verabreicht werden. Bevorzugt ist die Verwendung in Mischung mit geeigneten Hilfsstoffen oder Trägermaterial. Als Trägermaterial können bei Tierarzneimitteln die üblichen Futtermittelmischungen bzw. beim Menschen alle pharmakologisch verträglichen Trägermaterialien und/oder Hilfsstoffe verwendet werden.

Geeignete physiologisch verträgliche Salze der Verbindung der Formel I sind beispielsweise Alkali-, Erdalkali- und Ammoniumsalze einschließlich solcher von organischen Ammoniumbasen.

Die Anwendung betrifft auch pharmazeutische Zubereitungen der Chrysospermine.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische pflanzliche Öle, Polyethylenglykole und Lösungsmittel, wie etwa steriles Wasser, Alkohole, Glycerin und mehrwertige Alkohole genannt.

Eine erfindungsgemäße Verbindung kann außerdem in Verbindung mit weiteren Wirkstoffen, die eine identische oder verwandte Wirkungsweise haben, gegeben werden. Dies sind z.B. bei Abstoßungsreaktionen Antimicopathika, Thrombocytenaggregationshemmer, Analgetica und steroide oder nicht steroide Antiphlogistika.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis der Chrysospermine enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Schließlich können die Peptide der Formel I und/oder mindestens eines von deren physiologisch verträglichen Salzen bei der Herstellung der vorgenannten galenischen Zubereitungsformen auch zusammen mit anderen geeigneten Zubereitungsformen auch zusammen mit anderen geeigneten Wirkstoffen, beispielsweise anderen Antitumormitteln, Immunglobulinen, monoklonalen Antikörpern, immunstimulierenden Agenzien oder Antibiotika, formuliert werden. Diese Verbindungen können auch begleitend zu einer Strahlentheraphie verabreicht werden.

Die erfindungsgemäßen Arzneimittel werden dadurch hergestellt, daß man eine oder mehrere Verbindungen der Formel I mit üblichen Träger- sowie gegebenenfalls Zusatz- und/oder Hilfstoffe in die bzw. eine geeignete Darreichungsform bringt.

Ausführungsbeispiele

Die Erfindung wird durch die nachfolgenden Ausführungsbeispiele näher erläutert.

1.
a) Herstellung eines versporten Mycels des Stammes Apiocrea chrysosperma DSM 7444
Zur Herstellung eines versporten Myzels wird Apiocrea chrysosperma DSM 7444 15 Tage bei 25 °C auf Nährböden (4 % Malzextrakt, 0,4 % Hefeextrakt, 1,5 % Agar; Sterilisation 35 min bei 110 °C; pH 6,0), bebrütet.
b) Herstellung einer emersen Standkultur bzw. einer Vorkultur von Apiocrea chrysosperma DSM 7444 in Erlenmeyerkolben
Zur Anlage emerser Standkulturen werden in 500 ml Erlenmeyerkolben mit jeweils 100 ml flüssigem Nährmedium je 2 cm² große, mit Myzel bewachsene Agarstücke aus der im Beispiel 1a genannten Agarkultur eingeimpft. Das Nährmedium hat folgende Zusammensetzung (g/l): Malzextrakt 20; Glucose 10; Hefeextrakt 2; $(NH_4)_2HPO_4$ 5; destilliertes Wasser, pH 6,0 (Sterilisation 25 min bei 110 °C). Nach 15 Tagen bei 28 °C hat der Gehalt an Chrysosperminen in der Standkukltur den Maximalwert erreicht.
c) Herstellung von submersen Schüttelkulturen von Apiocrea chrysosperma DSM 7444
Es werden wie in Beispiel 1a und 1b in 500 ml Erlenmeyerkolben angelegte Kulturen in einem Schüttelinkubator bei 28 °C und 90 Umdrehungen/min 5 Tage lang geschüttelt. Von diesen Vorkulturen dienen je 5 ml Kultur zur Beimpfung der Hauptkultur. Diese erfolgt wiederum in 500 ml Erlenmeyerkolben mit 100 ml Malzextraktmedium wie unter Beispiel 1b beschrieben durch 7tägige Fermentation bei 28 °C und 90 Umdrehungen/min in einem Schüttelinkubator.
2. Herstellung und Aufreinigung der Peptidwirkstoffe Chrsospermine
15 Tage alte 5 l Standkulturen werden mit 3 x 1 l Ethylacetat jeweils über Nacht unter Rühren belassen. Die vereinigten Ethylacetatextrakte werden über Natriumsulfat getrocknet und bis auf ein Volumen von 50 ml eingeengt. Die Hauptmenge an Chrysosperminen wird anschließend durch Zugabe von 500 ml n-Hexan ausgefällt. Der nach dem Stehenlassen über Nacht bei 4 °C gebildete Niederschlag (ca. 750 mg) wird durch Säulenchromatographie an Kieselgel 60 (Porendurchmesser: 0,01 bis 0,02 mm) unter sequentieller Verwendung von Chloroform und Chloroform/Methanol (8:2; v/v) gereinigt. Die Chrysospermine enthaltenden Fraktionen werden durch ihre Inhibitionswirkung gegenüber Bacillus subtilis ATCC 6633 sowie durch ihre charakteristische rote Anfärbung im Dünnschichtchromatogramm durch eine Lösung von 2 % Vanillin in konz. Schwefelsäure erkannt. Ausbeute: 250 mg. Eine weitere Reinigung kann durch Wiederholung der Säulenchromatographie an Kieselgel 60 sowie durch Chromatographie an Sephadex LH-20 (Verwendung von Methanol als Laufmittel) erfolgen.

3. Chromatographische Auftrennung der Chrysospermine

Die weitere Reinigung und Auftrennung der Chrysospermine in die Einzelkomponenten A und B sowie C und D sowie in die Einzelkomponenten A bis D erfolgt durch wiederholte präparative Hochleistungsflüssigkeitschromatographie unter Verwendung von Kieselgel RP18 als Trägermaterial und Acetonitril/Wasser-Gemischen als Laufmittel.

10 mg des nach Beispiel 2 erhaltenen Produktes werden in 1 ml Methanol gelöst und auf eine RP18-HPLC-Säule (250 x 20 mm ID) aufgegeben. Die Elution erfolgt isokratisch mit Acetonitril/Wasser (50:50; v/v) bei einer Flußrate von 25 ml/min. Die getrennten Komponenten werden bei 220 nm detektiert.

In der ersten Trennphase erhält man die Teilkomplexe A + B (Retentionszeit 20 min) und C + D (Retentionszeit 30 min). Die getrennt aufgefangenen Eluate werden am Rotationsverdampfer bis zur wäßrigen Phase eingeengt und anschließend lyophylisiert.

Aus 100 mg eingesetztem Rohprodukt werden 12 mg Chrysospermin A + B und 58 mg Chrysospermin C + D erhalten.

Eine weitere Auftrennung in die Komponenten A bis D wird durch nochmalige präparative HPLC der Teilkomplexe A + B bzw. C + D unter den oben angeführten Bedingungen erreicht.

4. Physikalisch-chemische Eigenschaften der Chrysospermine

Aussehen: farblose kristalline Masse

Chromatographisches Verhalten (DC, Kieselgel-Alufolie Merck): $R_f$ 0,3 bis 0,4 (Chloroform/Methanol); 7 : 3)

Anfärbung durch chromatographische Nachweisreagentien:

2 % Vanillin/konz. Schwefelsäure: karminrote Anfärbung

Ninhydrin: schwache Anfärbung

Aminosäure im Hydrolysat: Ala, Aib, Leu, Gly, Glu, Pro, Ser, Phe sowie zwei weitere Komponenten (Iva, Trp-ol) (Nachweis durch GC-MS)

FAB-MS: Molpeaks m/z 1897 ($M^+ + H^+$); 1911 ($M^+ + H^+$), 1925 ($M^+ + H^+$).

5. Einfluß von Substanzen auf die mitogeninduzierte Proliferation von Lymphozyten der Milz

Es werden folgende Abkürzungen verwendet:

MW = Mittelwert

DIM = Dimensionierung

SD = Standardabweichung

Con A = Concanavalin A

LPS = Lipopolysaccharid

PWM = Pokeweedmitogen

SI = Stimulationsindex

Cryso = Crysospermin

Stl = Stammlösung

Grundmedium:

Clicks-/RPMI 1640 Medium (50:50) mit L-Glutamin ohne $NaHCO_3$ in Pulverform für 10 l (Seromed, Biochrom, Berlin, FRG), wird in 9 l Aqua bidest gelöst, und steril in Flaschen à 900 ml filtriert.

Waschmedium:

900 ml Grundmedium werden mit 9,5 ml 7,5 %iger Natriumhydrogencarbonat-Lösung und 5 ml HEPES (N-2-Hydroxyethyl-Piperazin-N-2-Ethanolsulfonsäure) (Gibco, Eggenstein, FRG) abgepuffert.

Gebrauchsmedium:

900 ml Grundmedium plus 19 ml $NaHCO_3$-Lösung (7,5 %; 10 ml HEPES-Lösung und 10 ml L-Glutamin-Lösung (200 mM)).

Medium für die mitogeninduzierte Lymphozytenproliferation:

Gebrauchsmedium wird mit 1 % hitzeinaktiviertem (30 min, 56 °C) foetalem Kälberserum (FCS) angesetzt.

Gewinnung und Aufarbeitung der Milzzellen für die mitogeninduzierte Lymphozytenproliferation

Die Mäuse werden durch Zervikaldislokation getötet und die Milzen steril entnommen. Auf einem sterilen Sieb mit einer Maschenweite von 80 "mash" werden die Milzen zerschnitten und mit dem Stempel einer Plastikspritze (10 ml) vorsichtig in eine Petrischale mit Gebrauchsmedium passiert. Zur Entfernung der Erythrozyten aus der Milzzellsuspension wird das Gemisch etwa 1 min, unter gelegentlichem Aufschütteln in hypotonischer, 0,17 M Ammoniumchloridlösung bei Raumtemperatur inkubiert. Die Erythrozyten werden dabei lysiert, während die Vitalität und Reaktivität der Lymphozyten nicht beeinflußt wird. Nach Zentrifugation (7 min/340 g) wird das Lysat verworfen, die Zellen zweimal gewaschen, im Testmedium aufgenommen und auf eine Zelldichte von $5 \times 10^6$ Zellen/ml eingestellt.

Testansatz:

Der Proliferationstest wird in Flachboden Mikrotiterplatten durchgeführt, das Gesamtvolumen pro Vertiefung beträgt 200 $\mu$l.

Formel 1 und Mitogene werden in Testmedium gelöst und auf die gewünschte Konzentration verdünnt. Je 50 $\mu$l Formelverdünnung und Mitogenverdünnung werden in die Vertiefungen vorgelegt (im Test werden die Werte vierfach bestimmt) und 100 $\mu$l der Milzzellsuspension ($5 \times 10^6$ Zellen/ml) zugegeben. Die Mitogene bewirken eine Proliferation verschiedener Lymphozytenpopulationen und werden in suboptimalen bis optimalen Stimulationskonzentrationen wie folgt eingesetzt:

| (Con A) Concavalin A: | 0,5-0,25 - 0,12 $\mu$g/ml |
|---|---|
| (LPS) Lipopolysaccharid: | 1,0-0,5 - 0,25 $\mu$g/ml |
| (PWM) Pokeweedmitogen: | 0,5-0,25 - 0,12 % Stammlösung. |

Die Platten werden verschloßen und 48 h bei 37 °C und 5 % $CO_2$ inkubiert. Die Proliferation wird dann durch radioaktive Markierung der DNA sich teilender Zellen bestimmt. Dazu werden den Vertiefungen 25 $\mu$l Tritium-Thymidin (Fa. Amersham) mit einer Aktivität von 0,25 $\mu$Ci/Vertiefung (spezifische Aktivität 29 Ci/mMol) zugegeben und für weitere 16 Stunden inkubiert.

Zur Auswertung des Testes werden die Platten über ein Zellerntegerät (Fa. Skatron) auf Glasfaserfilter geerntet, wobei nicht eingebautes Tritium-Thymidin in gesonderten Abfallflaschen aufgefangen wird und nur zellulär (in der DNA) gebundene Radioaktivität gemessen wird. Die Filter werden anschließend getrocknet, in Plastiktüten verschweißt und nach Zugabe von 10 ml Szintillator (Fa. Pharmacia) in Kasetten verschloßen. Die Messung erfolgt in einem Beta-Conter (Beta-Plate-System 1205 der Firma Wallac).

Berechnung:

Positivkontrolle: Lymphozyten der Milz mit den verschiedenen Mitogenen ohne Substanzzugabe.

Negativkontrolle: Lymphozyten der Milz in Medium (ohne Stimulanz) ohne Substanzzugabe.

Negativkontrolle der Präparatgruppen: Lymphozyten der Milz in Medium (ohne Stimulanz) plus Substanzzugabe.

Die % Änderung der Proliferation wird gegen die Positivkontrolle berechnet, wobei jeweils immer die gleiche Mitogenkonzentration des jeweiligen Mitogenes herangezogen wird.

Der Stimulationsindex gibt Aufschluß über die Stimulierbarkeit der Zellen gegen die Negativkontrolle der jeweiligen Präparatgruppe.

positiv Ko. SJ

| Mitogen | Dosis | Dim | n | MW | SD | SD in % | %-Ändg. | SI |
|---------|-------|-------|---|-------|------|---------|---------|------|
| Con A | 0.5 | µg/ml | 4 | 95347 | 7382 | 8 | 0 | 52.1 |
| Con A | 0.25 | µg/ml | 4 | 51945 | 6111 | 12 | 0 | 28.4 |
| Con A | 0.12 | µg/ml | 3 | 18408 | 3777 | 21 | 0 | 10.1 |
| LPS | 1.0 | µg/ml | 4 | 61044 | 2354 | 4 | 0 | 33.4 |
| LPS | 0.5 | µg/ml | 4 | 44816 | 1866 | 4 | 0 | 24.5 |
| LPS | 0.25 | µg/ml | 4 | 30710 | 2205 | 7 | 0 | 16.6 |
| PWM | 0.5 | % Stl | 4 | 28193 | 6757 | 24 | 0 | 15.4 |
| PWM | 0.25 | % Stl | 4 | 19343 | 3952 | 20 | 0 | 10.5 |
| PWM | 0.12 | % Stl | 4 | 17912 | 2073 | 12 | 0 | 9.8 |
| Medium | 0.0 | -- | 4 | 1829 | 601 | 33 | 0 | 1.0 |

| CsA 10 µg/ml | | | | | | | | |
|---------|-------|-------|---|-----|-----|---------|---------|------|
| Mitogen | Dosis | Dim | n | MW | SD | SD in % | %-Ändg. | SI |
| Con A | 0.5 | µg/ml | 4 | 236 | 174 | 74 | -100 | 14.7 |
| Con A | 0.25 | µg/ml | 4 | 229 | 145 | 64 | -100 | 16.4 |
| Con A | 0.12 | µg/ml | 4 | 36 | 10 | 29 | -100 | 2.2 |
| LPS | 1.0 | µg/ml | 4 | 23 | 2 | 9 | -100 | 1.4 |
| LPS | 0.5 | µg/ml | 4 | 30 | 6 | 19 | -100 | 1.9 |
| LPS | 0.25 | µg/ml | 4 | 19 | 5 | 29 | -100 | 1.2 |
| PWM | 0.5 | % Stl | 4 | 29 | 3 | 9 | -100 | 1.8 |
| PWM | 0.25 | % Stl | 4 | 24 | 1 | 6 | -100 | 1.5 |
| PWM | 0.12 | % Stl | 4 | 33 | 13 | 41 | -100 | 2.1 |
| Medium | 0.0 | -- | 4 | 16 | 4 | 23 | -99 | 1.0 |

| CsA 2 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 9733 | 373 | 4 | -90 | 28.5 |
| Con A | 0.25 | μg/ml | 4 | 3574 | 367 | 10 | -93 | 10.4 |
| Con A | 0.12 | μg/ml | 4 | 1405 | 127 | 9 | -92 | 4.1 |
| LPS | 1.0 | μg/ml | 3 | 9536 | 1110 | 12 | -84 | 27.9 |
| LPS | 0.5 | μg/ml | 4 | 5274 | 824 | 16 | -88 | 15.4 |
| LPS | 0.25 | μg/ml | 4 | 3080 | 64 | 2 | -90 | 9.0 |
| PWM | 0.5 | % Stl | 4 | 2354 | 182 | 8 | -92 | 6.9 |
| PWM | 0.25 | % Stl | 4 | 1778 | 55 | 3 | -91 | 5.2 |
| PWM | 0.12 | % Stl | 4 | 1478 | 194 | 13 | -92 | 4.3 |
| Medium | 0.0 | -- | 4 | 342 | 47 | 14 | -81 | 1.0 |

CsA 1 μg/ml

| Mitogen | Dosis | Dim | n | MW | SD | SD in % | %-Ändg. | SI |
|---|---|---|---|---|---|---|---|---|
| Con A | 0.5 | μg/ml | 4 | 11823 | 507 | 4 | -88 | 29.3 |
| Con A | 0.25 | μg/ml | 4 | 3673 | 652 | 18 | -93 | 9.1 |
| Con A | 0.12 | μg/ml | 4 | 1526 | 101 | 7 | -92 | 3.8 |
| LPS | 1.0 | μg/ml | 4 | 13284 | 1374 | 10 | -78 | 33.0 |
| LPS | 0.5 | μg/ml | 4 | 6982 | 699 | 10 | -84 | 17.3 |
| LPS | 0.25 | μg/ml | 4 | 4832 | 613 | 13 | -84 | 12.0 |
| PWM | 0.5 | % Stl | 4 | 2964 | 332 | 11 | -89 | 7.3 |
| PWM | 0.25 | % Stl | 4 | 2156 | 78 | 4 | -89 | 5.3 |
| PWM | 0.12 | % Stl | 4 | 1925 | 144 | 7 | -89 | 4.8 |
| Medium | 0.0 | -- | 4 | 403 | 19 | 5 | -78 | 1.0 |

| CsA 0.2 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 18370 | 579 | 3 | -81 | 35.5 |
| Con A | 0.25 | μg/ml | 4 | 5766 | 386 | 7 | -89 | 11.3 |
| Con A | 0.12 | μg/ml | 4 | 2010 | 80 | 4 | -89 | 3.9 |
| LPS | 1.0 | μg/ml | 4 | 23773 | 4940 | 21 | -61 | 45.9 |
| LPS | 0.5 | μg/ml | 4 | 16727 | 670 | 4 | -63 | 32.3 |
| LPS | 0.25 | μg/ml | 4 | 10136 | 336 | 3 | -67 | 19.6 |
| PWM | 0.5 | % Stl | 4 | 3928 | 306 | 8 | -86 | 7.6 |
| PWM | 0.25 | % Stl | 4 | 3085 | 257 | 8 | -84 | 6.0 |
| PWM | 0.12 | % Stl | 4 | 2430 | 270 | 11 | -86 | 4.7 |
| Medium | 0.0 | -- | 4 | 518 | 48 | 9 | -72 | 1.0 |

| CsA 0.1 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 20737 | 1584 | 8 | -78 | 37.7 |
| Con A | 0.25 | μg/ml | 4 | 5711 | 465 | 8 | -89 | 10.4 |
| Con A | 0.12 | μg/ml | 4 | 1938 | 213 | 11 | -89 | 3.5 |
| LPS | 1.0 | μg/ml | 4 | 29219 | 1486 | 5 | -52 | 53.1 |
| LPS | 0.5 | μg/ml | 4 | 18066 | 1086 | 6 | -60 | 32.8 |
| LPS | 0.25 | μg/ml | 4 | 11171 | 258 | 2 | -64 | 20.3 |
| PWM | 0.5 | % Stl | 4 | 4819 | 215 | 4 | -83 | 8.8 |
| PWM | 0.25 | % Stl | 4 | 3198 | 183 | 6 | -83 | 5.8 |
| PWM | 0.12 | % Stl | 4 | 2575 | 162 | 6 | -86 | 4.7 |
| Medium | 0.0 | -- | 4 | 550 | 35 | 6 | -70 | 1.0 |

| Cryso A/B 10 µg/ml | | | | | | | | |
|---|---|---|---|---|---|---|---|---|
| Mitogen | Dosis | Dim | n | MW | SD | SD in % | %-Ändg. | SI |
| Con A | 0.5 | µg/ml | 3 | 10830 | 3368 | 31 | -89 | 12.0 |
| Con A | 0.25 | µg/ml | 4 | 12891 | 548 | 4 | -75 | 14.2 |
| Con A | 0.12 | µg/ml | 4 | 8274 | 1192 | 14 | -55 | 9.0 |
| LPS | 1.0 | µg/ml | 3 | 6101 | 457 | 7 | -90 | 6.7 |
| LPS | 0.5 | µg/ml | 4 | 5697 | 231 | 4 | -87 | 6.3 |
| LPS | 0.25 | µg/ml | 4 | 4452 | 719 | 16 | -86 | 4.9 |
| PWM | 0.5 | % Stl | 4 | 5090 | 534 | 10 | -82 | 5.6 |
| PWM | 0.25 | % Stl | 4 | 5621 | 265 | 5 | -71 | 6.2 |
| PWM | 0.12 | % Stl | 3 | 3979 | 686 | 17 | -78 | 4.4 |
| Medium | 0.0 | -- | 4 | 905 | 311 | 34 | -50 | 1.0 |

| Cryso A/B 2 µg/ml | | | | | | | | |
|---|---|---|---|---|---|---|---|---|
| Mitogen | Dosis | Dim | n | MW | SD | SD in % | %-Ändg. | SI |
| Con A | 0.5 | µg/ml | 4 | 174598 | 2230 | 1 | 83 | 47.1 |
| Con A | 0.25 | µg/ml | 4 | 129118 | 2880 | 2 | 149 | 34.8 |
| Con A | 0.12 | µg/ml | 4 | 62937 | 11224 | 18 | 242 | 17.0 |
| LPS | 1.0 | µg/ml | 4 | 74404 | 1886 | 3 | 22 | 20.1 |
| LPS | 0.5 | µg/ml | 4 | 58285 | 1669 | 3 | 30 | 15.7 |
| LPS | 0.25 | µg/ml | 4 | 47409 | 3056 | 6 | 54 | 12.8 |
| PWM | 0.5 | % Stl | 4 | 51533 | 3863 | 7 | 83 | 13.9 |
| PWM | 0.25 | % Stl | 4 | 43847 | 1837 | 4 | 127 | 11.8 |
| PWM | 0.12 | % Stl | 4 | 32537 | 1237 | 4 | 82 | 8.8 |
| Medium | 0.0 | -- | 4 | 3708 | 264 | 7 | 103 | 1.0 |

| Cryso A/B 1 µg/ml | | | | | | | | |
|---|---|---|---|---|---|---|---|---|
| Mitogen | Dosis | Dim | n | MW | SD | SD in % | %-Ändg. | SI |
| Con A | 0.5 | µg/ml | 4 | 150893 | 6464 | 4 | 58 | 36.7 |
| Con A | 0.25 | µg/ml | 4 | 119556 | 1975 | 2 | 130 | 29.1 |
| Con A | 0.12 | µg/ml | 4 | 60761 | 3799 | 6 | 230 | 14.8 |
| LPS | 1.0 | µg/ml | 4 | 61155 | 8047 | 13 | 0 | 14.9 |
| LPS | 0.5 | µg/ml | 4 | 54958 | 2463 | 4 | 23 | 13.4 |
| LPS | 0.25 | µg/ml | 4 | 44582 | 1861 | 4 | 45 | 10.8 |
| PWM | 0.5 | % Stl | 4 | 53906 | 3318 | 6 | 91 | 13.1 |
| PWM | 0.25 | % Stl | 4 | 42879 | 1725 | 4 | 122 | 10.4 |
| PWM | 0.12 | % Stl | 4 | 31055 | 1013 | 3 | 73 | 7.5 |
| Medium | 0.0 | -- | 4 | 4114 | 485 | 12 | 125 | 1.0 |

| Cryso A/B 0.2 µg/ml | | | | | | | | |
|---|---|---|---|---|---|---|---|---|
| Mitogen | Dosis | Dim | n | MW | SD | SD in % | %-Ändg. | SI |
| Con A | 0.5 | µg/ml | 4 | 168295 | 4045 | 2 | 77 | 50.4 |
| Con A | 0.25 | µg/ml | 4 | 124940 | 3446 | 3 | 141 | 37.4 |
| Con A | 0.12 | µg/ml | 4 | 67198 | 8378 | 12 | 265 | 20.1 |
| LPS | 1.0 | µg/ml | 4 | 70965 | 4476 | 6 | 16 | 21.2 |
| LPS | 0.5 | µg/ml | 4 | 59873 | 1326 | 2 | 34 | 17.9 |
| LPS | 0.25 | µg/ml | 4 | 44695 | 660 | 1 | 46 | 13.4 |
| PWM | 0.5 | % Stl | 4 | 49539 | 1364 | 3 | 76 | 14.8 |
| PWM | 0.25 | % Stl | 4 | 41145 | 1357 | 3 | 113 | 12.3 |
| PWM | 0.12 | % Stl | 4 | 31056 | 1722 | 6 | 73 | 9.3 |
| Medium | 0.0 | -- | 4 | 3338 | 280 | 8 | 83 | 1.0 |

| Cryso A/B 0.1 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 167376 | 6191 | 4 | 76 | 44.7 |
| Con A | 0.25 | μg/ml | 4 | 134097 | 6943 | 5 | 158 | 35.8 |
| Con A | 0.12 | μg/ml | 4 | 62049 | 2175 | 4 | 237 | 16.5 |
| LPS | 1.0 | μg/ml | 4 | 68121 | 8000 | 12 | 12 | 18.2 |
| LPS | 0.5 | μg/ml | 4 | 61682 | 2884 | 5 | 38 | 16.5 |
| LPS | 0.25 | μg/ml | 4 | 44654 | 1859 | 4 | 45 | 11.9 |
| PWM | 0.5 | % Stl | 4 | 52551 | 1348 | 3 | 86 | 14.0 |
| PWM | 0.25 | % Stl | 4 | 41341 | 473 | 1 | 114 | 11.0 |
| PWM | 0.12 | % Stl | 4 | 30586 | 219 | 1 | 71 | 8.2 |
| Medium | 0.0 | -- | 4 | 3747 | 354 | 9 | 105 | 1.0 |

| Cryso C/D 10 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 219 | 29 | 13 | -100 | 4.7 |
| Con A | 0.25 | μg/ml | 4 | 171 | 35 | 20 | -100 | 3.7 |
| Con A | 0.12 | μg/ml | 4 | 85 | 13 | 15 | -100 | 1.8 |
| LPS | 1.0 | μg/ml | 4 | 47 | 4 | 8 | -100 | 1.0 |
| LPS | 0.5 | μg/ml | 4 | 100 | 37 | 37 | -100 | 2.2 |
| LPS | 0.25 | μg/ml | 4 | 66 | 20 | 30 | -100 | 1.4 |
| PWM | 0.5 | % Stl | 4 | 87 | 17 | 19 | -100 | 1.9 |
| PWM | 0.25 | % Stl | 4 | 61 | 4 | 7 | -100 | 1.3 |
| PWM | 0.12 | % Stl | 4 | 67 | 14 | 21 | -100 | 1.5 |
| Medium | 0.0 | -- | 4 | 46 | 12 | 25 | -98 | 1.0 |

| Cryso C/D 2 μg/ml | | | | | | | | |
|---|---|---|---|---|---|---|---|---|
| Mitogen | Dosis | Dim | n | MW | SD | SD in % | %-Andg. | SI |
| Con A | 0.5 | μg/ml | 4 | 168994 | 2499 | 1 | 77 | 46.1 |
| Con A | 0.25 | μg/ml | 4 | 127101 | 3894 | 3 | 145 | 34.7 |
| Con A | 0.12 | μg/ml | 4 | 55318 | 1539 | 3 | 201 | 15.1 |
| LPS | 1.0 | μg/ml | 3 | 46853 | 1529 | 3 | -23 | 12.8 |
| LPS | 0.5 | μg/ml | 4 | 34354 | 892 | 3 | -23 | 9.4 |
| LPS | 0.25 | μg/ml | 4 | 26359 | 1482 | 6 | -14 | 7.2 |
| PWM | 0.5 | % Stl | 4 | 31481 | 815 | 3 | 12 | 8.6 |
| PWM | 0.25 | % Stl | 4 | 26621 | 3468 | 13 | 38 | 7.3 |
| PWM | 0.12 | % Stl | 4 | 19632 | 690 | 4 | 10 | 5.4 |
| Medium | 0.0 | -- | 4 | 3663 | 319 | 9 | 100 | 1.0 |

| Cryso C/D 1 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 186499 | 5377 | 3 | 96 | 46.8 |
| Con A | 0.25 | μg/ml | 4 | 131375 | 1171 | 1 | 153 | 33.0 |
| Con A | 0.12 | μg/ml | 4 | 62219 | 4252 | 7 | 238 | 15.6 |
| LPS | 1.0 | μg/ml | 4 | 66784 | 8884 | 13 | 9 | 16.8 |
| LPS | 0.5 | μg/ml | 4 | 52076 | 1741 | 3 | 16 | 13.1 |
| LPS | 0.25 | μg/ml | 4 | 38876 | 1957 | 5 | 27 | 9.8 |
| PWM | 0.5 | % Stl | 4 | 57400 | 12906 | 22 | 104 | 14.4 |
| PWM | 0.25 | % Stl | 4 | 37675 | 1814 | 5 | 95 | 9.5 |
| PWM | 0.12 | % Stl | 4 | 31638 | 2867 | 9 | 77 | 7.9 |
| Medium | 0.0 | -- | 4 | 3981 | 462 | 12 | 118 | 1.0 |

| Cryso C/D 0.2 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 174176 | 1882 | 1 | 83 | 37.9 |
| Con A | 0.25 | μg/ml | 4 | 131338 | 3245 | 2 | 153 | 28.6 |
| Con A | 0.12 | μg/ml | 4 | 62224 | 3767 | 6 | 238 | 13.5 |
| LPS | 1.0 | μg/ml | 4 | 67510 | 8444 | 13 | 11 | 14.7 |
| LPS | 0.5 | μg/ml | 4 | 58617 | 1115 | 2 | 31 | 12.8 |
| LPS | 0.25 | μg/ml | 4 | 47545 | 1183 | 2 | 55 | 10.3 |
| PWM | 0.5 | % Stl | 4 | 56805 | 2137 | 4 | 101 | 12.3 |
| PWM | 0.25 | % Stl | 4 | 43419 | 1990 | 5 | 124 | 9.4 |
| PWM | 0.12 | % Stl | 4 | 30425 | 2844 | 9 | 70 | 6.6 |
| Medium | 0.0 | -- | 4 | 4596 | 188 | 4 | 151 | 1.0 |

| Cryso C/D 0.1 μg/ml | | | | | | | | |
|---|---|---|---|---|---|---|---|---|
| Mitogen | Dosis | Dim | n | MW | SD | SD in % | %-Ändg. | SI |
| Con A | 0.5 | μg/ml | 4 | 174085 | 1944 | 1 | 83 | 40.5 |
| Con A | 0.25 | μg/ml | 4 | 122841 | 1477 | 1 | 136 | 28.6 |
| Con A | 0.12 | μg/ml | 4 | 61752 | 5629 | 9 | 235 | 14.4 |
| LPS | 1.0 | μg/ml | 4 | 70069 | 2049 | 3 | 15 | 16.3 |
| LPS | 0.5 | μg/ml | 4 | 59291 | 3267 | 6 | 32 | 13.8 |
| LPS | 0.25 | μg/ml | 4 | 42247 | 1788 | 4 | 38 | 9.8 |
| PWM | 0.5 | % Stl | 4 | 55930 | 2910 | 5 | 98 | 13.0 |
| PWM | 0.25 | % Stl | 4 | 47362 | 1848 | 4 | 145 | 11.0 |
| PWM | 0.12 | % Stl | 4 | 30606 | 5959 | 19 | 71 | 7.1 |
| Medium | 0.0 | -- | 4 | 4295 | 271 | 6 | 135 | 1.0 |

6. Tumorzellproliferation (in vitro):

Der Proliferationstest wird in Rundboden Mikrotiterplatten durchgeführt. Tumorzellen, ursprünglich bezogen von American Type Culture Collection (ATCC), werden in einer permanenten Stammhaltung in serumfreien Medium (CG-Medium von Vitromex) gezüchtet und in ihrer logarithmischen Zellführung zum Test herangezogen. Die folgenden Tumorzellinien werden routinemäßig verwendet:

1) A 20.2 J = Plasmazytom
2) 20-10-5S = Hybridom
3) EL4 = T-Helferzell-Lymphom
4) K562 = undifferenzierte myelomonozytäre Linie

Die Zellen werden in serumfreiem CG-Medium auf eine Zelldichte von $4 \times 10^4$ Zellen/ml eingestellt und 100 μl/Vertiefung pipettiert. Formel 1 wird in CG-Medium gelöst, auf die gewünschte Konzentration herunter verdünnt und 100 μl zu den Zellen zugegeben (Gesamtvolumen 200 μl mit $4 \times 10^3$ Zellen). Die jeweiligen Werte werden auch durch 4fach Bestimmung ermittelt.

Nach einer 48stündigen Inkubation wird die Proliferation der Zellen durch Einbau von radioaktivem Tritium-Thymidin, analog wie unter Ziffer 5 beschrieben, ermittelt.

Berechnung:

Positivkontrolle: Zellen jeder Zellinie werden in Medium inkubiert = normale Proliferation.

%Änderung der Proliferation der einzelenen Präparatkonzentrationen werden gegen die Positivkontrolle berechnet.

| Zellinie: EL 4 | | | | | | |
|---|---|---|---|---|---|---|
| Präparat | µg/ml | n | MW | SD | SD in % | %-Änderung |
| Cyclosporin A | 10.000 | 4 | 17 | 5 | 29 | -100 |
| | 5.000 | 4 | 14 | 1 | 9 | -100 |
| | 2.500 | 4 | 22 | 8 | 35 | -100 |
| | 1.000 | 4 | 30 | 6 | 21 | -100 |
| | 0.500 | 4 | 2608 | 409 | 16 | -97 |
| | 0.100 | 4 | 27333 | 1725 | 6 | -73 |
| Chrysospermin A/B | 10.000 | 4 | 27 | 23 | 85 | -100 |
| | 5.000 | 4 | 65835 | 4013 | 6 | -35 |
| | 2.500 | 4 | 87715 | 3042 | 3 | -14 |
| | 1.000 | 4 | 90812 | 2784 | 3 | -11 |
| | 0.500 | 4 | 92122 | 5057 | 5 | -10 |
| | 0.100 | 4 | 93904 | 4574 | 5 | -8 |
| Chrysospermin C/D | 10.000 | 4 | 22 | 4 | 17 | -100 |
| | 5.000 | 4 | 25 | 9 | 35 | -100 |
| | 2.500 | 4 | 72404 | 3527 | 5 | -29 |
| | 1.000 | 4 | 90971 | 2212 | 2 | -11 |
| | 0.500 | 4 | 90831 | 574 | 1 | -11 |
| | 0.100 | 4 | 91906 | 4771 | 5 | -10 |

| Zellinie: K 562 | | | | | | |
|---|---|---|---|---|---|---|
| Präparat | µg/ml | n | MW | SD | SD in % | %-Änderung |
| Cyclosporin A | 10.000 | 4 | 33 | 6 | 17 | -100 |
| | 5.000 | 4 | 1027 | 67 | 7 | -99 |
| | 2.500 | 4 | 19191 | 978 | 5 | -85 |
| | 1.000 | 4 | 83706 | 4384 | 5 | -36 |
| | 0.500 | 4 | 100838 | 5169 | 5 | -23 |
| | 0.100 | 4 | 126497 | 6596 | 5 | -4 |
| Chrysospermin A/B | 10.000 | 4 | 1560 | 403 | 26 | -99 |
| | 5.000 | 4 | 18441 | 1733 | 9 | -86 |
| | 2.500 | 4 | 52008 | 1459 | 3 | -60 |
| | 1.000 | 4 | 123531 | 2903 | 2 | -6 |
| | 0.500 | 4 | 124321 | 5204 | 4 | -5 |
| | 0.100 | 4 | 130717 | 6127 | 5 | -1 |
| Chrysospermin C/D | 10.000 | 4 | 19 | 3 | 18 | -100 |
| | 5.000 | 4 | 1143 | 265 | 23 | -99 |
| | 2.500 | 4 | 16716 | 1384 | 8 | -87 |
| | 1.000 | 4 | 112024 | 8598 | 8 | -15 |
| | 0.500 | 4 | 124858 | 2260 | 2 | -5 |
| | 0.100 | 4 | 130525 | 5893 | 5 | -1 |
| CT = 2501 = | | | | | | |

| Zellinie: A20.2.J | | | | | | |
|---|---|---|---|---|---|---|
| Präparat | μg/ml | n | MW | SD | SD in % | %-Änderung |
| Cyclosporin A | 10.000 | 4 | 17 | 3 | 18 | -100 |
| | 5.000 | 4 | 31 | 9 | 28 | -100 |
| | 2.500 | 4 | 1495 | 436 | 29 | -99 |
| | 1.000 | 4 | 24936 | 1278 | 5 | -85 |
| | 0.500 | 4 | 58911 | 1357 | 2 | -64 |
| | 0.100 | 4 | 136012 | 4587 | 3 | -16 |
| Chrysospermin A/B | 10.000 | 4 | 35 | 9 | 25 | -100 |
| | 5.000 | 4 | 39511 | 3265 | 8 | -76 |
| | 2.500 | 4 | 148134 | 5418 | 4 | -8 |
| | 1.000 | 4 | 156242 | 4857 | 3 | -3 |
| | 0.500 | 4 | 157810 | 4028 | 3 | -2 |
| | 0.100 | 4 | 154432 | 6877 | 4 | -5 |
| Chrysospermin C/D | 10.000 | 4 | 31 | 9 | 31 | -100 |
| | 5.000 | 4 | 74 | 41 | 55 | -100 |
| | 2.500 | 4 | 80974 | 2016 | 2 | -50 |
| | 1.000 | 4 | 160019 | 5467 | 3 | -1 |
| | 0.500 | 4 | 156971 | 8541 | 5 | -3 |
| | 0.100 | 4 | 152791 | 8817 | 6 | -6 |

| Zellinie: 20-10-5S | | | | | | |
|---|---|---|---|---|---|---|
| Präparat | μg/ml | n | MW | SD | SD in % | %-Änderung |
| Cyclosporin A | 10.000 | 4 | 20 | 5 | 28 | -100 |
| | 5.000 | 4 | 58 | 51 | 88 | -100 |
| | 2.500 | 4 | 19 | 10 | 54 | -100 |
| | 1.000 | 4 | 44 | 46 | 105 | -100 |
| | 0.500 | 4 | 29 | 17 | 57 | -100 |
| | 0.100 | 4 | 33784 | 5010 | 15 | -42 |
| Chrysospermin A/B | 10.000 | 4 | 16 | 5 | 29 | -100 |
| | 5.000 | 4 | 20865 | 5059 | 24 | -64 |
| | 2.500 | 4 | 62699 | 6069 | 10 | +8 |
| | 1.000 | 4 | 62344 | 2851 | 5 | +7 |
| | 0.500 | 4 | 60208 | 1632 | 3 | +4 |
| | 0.100 | 4 | 65147 | 2830 | 4 | +12 |
| Chrysospermin C/D | 10.000 | 4 | 16 | 3 | 20 | -100 |
| | 5.000 | 4 | 28 | 19 | 67 | -100 |
| | 2.500 | 4 | 47995 | 1248 | 3 | -17 |
| | 1.000 | 4 | 60538 | 1657 | 3 | 4 |
| | 0.500 | 4 | 61930 | 4507 | 7 | 7 |
| | 0.100 | 4 | 59966 | 1819 | 3 | 3 |

7. Beeinflussung der Interleukin 2/3-Produktion durch CSA Crysospermin A/B / C/D

Die Methode zur Gewinnung der Lymphozyten ist unter Pkt. 5 beschrieben.

Ansatz zur Interleukin-Gewinnung: Makroplatten mit jeweils 12 Vertiefungen; $5 \times 10^6$ NMRJ-Milzzellen in 4 ml CTL-Medium (= Cytoxisches T-Lymphozyten-Medium) + 2 μl/ml Concanavalin A bzw. ohne Stimulanz und CSA (Cyclosporin A), Crysospermin A/B bzw. C/D in verschiedenen Konzentrationen. 24 Std. Stimulation; Überstand wird zellfrei gewonnen.

Cytotox.-T-Lymph-Medium: CIR-Medium (Fa. Biochrom) + 5 % foetales Kälberserum + 1 % NEA (= nicht essentielle Aminosäuren) + 1 % Pyruvat + ME ($\beta$-Mercaptoethanol $5 \times 10^{-5}$ ml); alles von (Fa. Gibco)

Dieser Überstand wird auf Interleukin-2/-3 abhängig wachsende Zellinien gegeben und der Gehalt an Interleukin anhand der Proliferation gemessen.

CTLL-Zellen → IL-2-Nachweis

DA 1-Zellen → IL-3-Nachweis

Ansatz zur Testung des Interleukin Gehaltes des Überstands:

$4 \times 10^3$ der obengenannten Zellen / 100 $\mu$l

+ 100 $\mu$l Überstand = 50%}

+ 50 $\mu$l Überstand = 25 %} Gesamtvolumen 200 $\mu$l pro Vertiefung

+ 25 $\mu$l Überstand = 12,5 %}

+ 12,5 $\mu$l Überstand = 6,25 %}

Die Platten werden 48 Stunden inkubiert und die Proliferation der Zellen durch Einbau von radioaktivem Tritium-Thymidin, analog wie unter Pkt. 5 beschrieben, ermittelt.

Berechnung:

Milzzellen ohne Substanz mit Concanavalin A als Stimulanz produzieren Interleukin 2 und 3. Dieser Überstand wird als positive Referenzkontrolle herangezogen und die jeweiligen Verdünnungen der Überstände der einzelnen Präparatkonzentrationen dagegen verglichen.

* Berechnung auf Signifikanz mit $p \leq 0.5$.

Testinterner Standard: Zur Überprüfung wie hoch der Gehalt an Interleukin 2 bzw. 3 bei den einzelnen Gruppen ist, werden Interleukin 2 bzw. 3 Kontrollansätze bekannter Konzentrationen mitgetestet. Mikroskopische Beurteilung nach 20 Std.

50 $\mu$g/ml und 20 $\mu$g/ml aller 3 Substanzen 100 % tote Zellen

| Interleukin 2-Kontrolle: | | | |
|---|---|---|---|
| | | | Counts |
| (1) | 80 $\mu$g/ml | 200 U/ml | 233.430 |
| (2) | 40 $\mu$g/ml | 100 U/ml | 229.583 |
| (3) | 20 $\mu$g/ml | 50 U/ml | 230.215 |
| (4) | 10 $\mu$g/ml | 25 U/ml | 225.827 |
| (5) | 5 $\mu$g/ml | 12,5 U/ml | 183.303 |
| (6) | 2,5 $\mu$g/ml | 6,25 U/ml | 78.836 |
| (7) | 1,25 $\mu$g/ml | 3,125 U/ml | 42.751 |
| (8) | Medium | Medium | 43 |

| Interleukin 3-Kontrolle: | | |
|---|---|---|
| (1) | 20 % | 44.525 |
| (2) | 10 % | 38.252 |
| (3) | 5 % | 33.018 |
| (4) | 2,5 % | 24.826 |
| (5) | 1,25 % | 12.102 |
| (6) | 0,6 . 125 % | 5.305 |
| (7) | 0,3 . 2 % | 2.580 |
| (8) | Medium | 20 |

## Interleukin-3

| Cyclosporin A | Kontrolle | 50 μg/ml | 20 μg/ml | 10 μg/ml | 2 μg/ml | 0,2 μg/ml |
|---|---|---|---|---|---|---|
| 50 % | 9798 | Ø | Ø | Ø | Ø | Ø |
| 25 % | 4300 | Ø | Ø | Ø | Ø | Ø |
| 12,5 % | 1218 | Ø | Ø | Ø | Ø | Ø |
| 6,25 % | 181 | Ø | Ø | Ø | Ø | Ø |

## Interleukin-3

| Crysospermin A/B | Kontrolle | 50 μg/ml | 20 μg/ml | 10 μg/ml | 2 μg/ml | 0,2 μg/ml |
|---|---|---|---|---|---|---|
| 50 % | 9798 | Ø | Ø | 9864 | 15671* 11,0 59,8 | 14766 * |
| 25 % | 4300 | Ø | Ø | 3468 | 5969 * | 5568 * |
| 12,5 % | 1218 | Ø | Ø | 685 | 2403 * | 2165 * |
| 6,25 % | 181 | Ø | Ø | 42 | 515 * | 498 * |

EP 0 622 375 A1

Interleukin-3

| CID | Kontrolle | 50 µg/ml | 20 µg/ml | 10 µg/ml | 2 µg/ml | 0,2 µg/ml |
|---|---|---|---|---|---|---|
| 50 % | 9798 | Ø | Ø | 321 | 15121 * | 15613 * |
| 25 % | 4300 | Ø | Ø | Ø | 5907 * | 5817 * |
| 12,5 % | 1218 | Ø | Ø | Ø | 2170 * | 1863 * |
| 6,25 % | 181 | Ø | Ø | Ø | 519 * | 292 * |

Interluekin-2

| CSA | Kontrolle | 50 µg/ml | 20 µg/ml | 10 µg/ml | 2 µg/ml | 0,2 µg/ml |
|---|---|---|---|---|---|---|
| 50 % | 73812 | 2090 | 162 | Ø | Ø | Ø |
| 25 % | 30347 | 982 | 153 | Ø | Ø | Ø |
| 12,5 % | 2766 | Ø | Ø | Ø | Ø | Ø |
| 6,25 % | 270 | Ø | Ø | Ø | Ø | Ø |

Interleukin-2

| AIB | Kontrolle | 50 µg/ml | 20 µg/ml | 10 µg/ml | 2 µg/ml | 0,2 µg/ml |
|---|---|---|---|---|---|---|
| 50 % | 73812 | Ø | 3663 | 70918 | 85632 | 83622 |
| 25 % | 30347 | Ø | 796 | 45892 * | 42054 * | 72513 * |
| 12,5 % | 2766 | Ø | 693 | 13617 * | 5821 * | 9610 * |
| 6,25 % | 270 | Ø | Ø | 458 | 347 | 419 |

Interleukin-2

| CID | Kontrolle | 50 µg/ml | 20 µg/ml | 10 µg/ml | 2 µg/ml | 0,2 µg/ml |
|---|---|---|---|---|---|---|
| 50 % | 73812 | Ø | Ø | 19483 | 92026 * | 81028 |
| 25 % | 30347 | Ø | Ø | 11524 | 53193 * | 52904 * |
| 12,5 % | 2766 | Ø | Ø | 696 | 8812 * | 19025 * |
| 6,25 % | 170 | Ø | Ø | 165 | 614 | 4514 * |

8. Chemilumineszens in vitro

Teflonbeutel-Anzuchtmethode:

Von 6-10 Wochen alten NMRI-Mäusen werden die beiden Femur unter sterilen Bedingungen möglichst muskelfrei entnommen. Das Knochenmark wird mit 10 ml Gebrauchsmedium (siehe Pkt. 5) durch Einführen einer Kanüle an einer Seite des Knochens herausgespült und durch mehrmaliges Aufziehen in die Spritze in Einzelzellen zerlegt.

Teflonfolie (Biofolie 25, Fa. Heraeus) wird mit der hydrophilen Seite nach innen in Beuteln des Formates 5 cm x 30 cm zurechtgeschweißt und bei 121°C und 1,1 bar für 20 Minuten dampfsterilisiert. In diese sterilen Beutel werden 4 x 10⁶ Stammzellen in 60 ml Gebrauchsmedium (siehe Pkt. 5) mit 20 % FCS und 30 % L929-Überstand (als Quelle für Colony-Stimulating-Faktor 1 CSF1 zur Ausdifferenzierung zu Makrophagen) gegeben und für 8 Tage bei 37°C und 5 % $CO_2$ inkubiert.

Makrophagen werden mittels der Teflonbeutelmethode aus einer NMRI-Maus hochgezogen. Den Zellen wird 8 Tage lang Zeit zur Ausdifferenzierung gelassen. Nachfolgend werden sie mit Hilfe von Giemsa angefärbt, um die Anzahl der Makrophage bestimmen zu können.

Ansatz:

**200 µl Substanz der jeweiligen Konzentration**

**350 µl RPMI-Medium 1640 (Biochrom) ohne Phenolrot**

**100 µl Luminol (200 µM)**

**100 µl Makrophagen (2.5 x 10⁶/ml)**

**250 µl PMA (3.5 µM = Phorbolmyristinacetat)**

**1000 µl ≙ PMA 0.875 µM }** werden mit 2.5 x 10⁶ Makrophagen

**≙ Luminol 20 µM }** inkubiert

Chemilumineszens wird gemessen im Picolite 6500 (von Packard, USA); Photonen pro 10 Sekunden.

Durch Zugabe von PMA werden die Makrophagen aktiviert.

2 Ansätze pro Präparatkonzentration (Ergebnisse = X = Mittelwerte)

Ohne Vorinkubation:    Testsubstanz + PMA und nachfolgend messen

1 Std. Vorinkubation:    Testsubstanz 1 Std. bei 37°C danach PMA und nachfolgend messen

| | ohne Vorinkubation | 1 Std. Vorinkubation |
|---|---|---|
| Minuten | Positivkontrolle | Positivkontrolle |
| 0' | 23835 | 34360 |
| 2' | 92600 | 95605 |
| 4' | 74465 | 74940 |
| 6' | 66300 | 55480 |
| 8' | 57955 | 47450 |
| 10' | 51580 | 42210 |
| 12' | 45850 | 36795 |
| 14' | 39460 | 32745 |
| 16' | 35065 | 30050 |
| 18' | 33790 | 29225 |

|  | ohne Vorinkubation | | | 1 Std. Vorinkubation | | |
|---|---|---|---|---|---|---|
| Minuten | CsA 50 μg/ml | A/B 50 μg/ml | C/D 50 μg/ml | CsA 50 μg/ml | A/B 50 μg/ml | C/D 50 μg/m |
| 0' | 16425 | 14910 | 16155 | 14680 | 18405 | 22425 |
| 2' | 13869 | 45610 | 25090 | 14405 | 42450 | 30470 |
| 4' | 15784 | 29505 | 22215 | 18630 | 27295 | 21060 |
| 6' | 21995 | 24615 | 21090 | 21315 | 23805 | 18330 |
| 8' | 23950 | 22760 | 22085 | 21715 | 21115 | 17660 |
| 10' | 24820 | 20460 | 22175 | 21345 | 18530 | 17610 |
| 12' | 23425 | 17564 | 21035 | 20455 | 17715 | 17600 |
| 14' | 22400 | 17505 | 20070 | 20335 | 17985 | 16965 |
| 16' | 22625 | 15169 | 18255 | 21340 | 16245 | 17080 |
| 18' | 21930 | 13835 | 17309 | 21055 | 15835 | 15765 |

|  | ohne Vorinkubation | | | 1 Std. Vorinkubation | | |
|---|---|---|---|---|---|---|
| Minuten | CsA 10 μg/ml | A/B 10 μg/ml | C/D 10 μg/ml | CsA 10 μg/ml | A/B 10 μg/ml | C/D 10 μg/ml |
| 0' | 11051 | 31205 | 27095 | 10960 | 46290 | 44245 |
| 2' | 17060 | 111849 | 97595 | 15675 | 129315 | 124995 |
| 4' | 36365 | 81980 | 76105 | 37100 | 84195 | 89300 |
| 6' | 30455 | 77230 | 69970 | 30080 | 72935 | 77950 |
| 8' | 29755 | 70430 | 64035 | 26065 | 62390 | 73230 |
| 10' | 28555 | 66800 | 64465 | 25060 | 57820 | 70070 |
| 12' | 27960 | 61285 | 61940 | 24075 | 52245 | 69655 |
| 14' | 26665 | 56980 | 59810 | 46040 | 47335 | 63110 |
| 16' | 25605 | 515151 | 58130 | 41630 | 43650 | 59310 |
| 18' | 24175 | 46940 | 54050 | 20990 | 42065 | 56880 |

| | ohne Vorinkubation | | | 1 Std. Vorinkubation | | |
|---|---|---|---|---|---|---|
| Minuten | CsA 5 $\mu$g/ml | A/B 5 $\mu$g/ml | C/D 5 $\mu$g/ml | CsA 5 $\mu$g/ml | A/B 5 $\mu$g/ml | C/D 5 $\mu$g/ml |
| 0' | 13755 | 22210 | 24520 | 10270 | 53265 | 38675 |
| 2' | 35215 | 106665 | 105205 | 26905 | 131975 | 120450 |
| 4' | 43660 | 77080 | 76620 | 44155 | 97245 | 90695 |
| 6' | 37095 | 72010 | 75345 | 34020 | 83945 | 79420 |
| 8' | 35505 | 69565 | 70915 | 29475 | 77030 | 74430 |
| 10' | 33465 | 67640 | 68440 | 28080 | 69515 | 72315 |
| 12' | 30935 | 62835 | 67550 | 25685 | 64360 | 65520 |
| 14' | 29235 | 58885 | 65280 | 23775 | 57995 | 63277 |
| 16' | 27800 | 55405 | 58435 | 22855 | 56035 | 59030 |
| 18' | 25775 | 52860 | 54015 | 21225 | 52560 | 57400 |

| | ohne Vorinkubation | | | 1 Std. Vorinkubation | | |
|---|---|---|---|---|---|---|
| Minuten | CsA 1 $\mu$g/ml | A/B 1 $\mu$g/ml | C/D 1 $\mu$g/ml | CsA 1 $\mu$g/ml | A/B 1 $\mu$g/ml | C/D 1 $\mu$g/ml |
| 0' | 12100 | 16595 | 19710 | 24570 | 16550 | 23400 |
| 2' | 60880 | 948255 | 97040 | 75020 | 110160 | 113455 |
| 4' | 47915 | 69665 | 71185 | 59415 | 75800 | 76945 |
| 6' | 39600 | 64225 | 64900 | 44310 | 61185 | 60380 |
| 8' | 38210 | 61365 | 57805 | 37860 | 58505 | 54930 |
| 10' | 36230 | 58231 | 51410 | 33715 | 55910 | 47705 |
| 12' | 32615 | 54115 | 46950 | 31295 | 51265 | 41055 |
| 14' | 28520 | 49605 | 61420 | 27260 | 46470 | 36430 |
| 16' | 25930 | 44700 | 36790 | 25870 | 44145 | 33485 |
| 18' | 24570 | 39165 | 32015 | 24200 | 40125 | 31520 |

9. MLR one way (Mixed Lymphocyte Reaction) NMRI-Milzzellen gegen EL4 Tumorzellen

Mitomycin C-Behandlung: (EL4 können nicht mehr proliferieren)

7,75 x $10^6$ EL4-Zellen werden mit 30 $\mu$g Mitomycin C in 2 ml $\triangleq$ (1 x $10^6$ Zellen + 3,9 $\mu$g Mitomycin in 258 $\mu$l Medium) für 1 Std. im $H_2$O-Bad bei 37°C inkubiert und nachfolgend für 30 Minuten in frischem Medium im $H_2$O-Bad ausschwitzen lassen. Zuletzt werden die Zellen zweimal mit Grundmedium gewaschen.

Ansatz:

100 $\mu$l NMRI-Milzzellen 5 x $10^5$/Vertiefung

50 $\mu$l EL4-Zellen (2.3 x $10^4$) bzw. (4.7 x $10^4$) pro Vertiefung

50 $\mu$l Substanz (4 x konz.)

in CTL-Medium (CTL-Medium mit 5 % foetalem Kälberserum; 1 % NEA; 1 % Pyruvat + ME) in Flachbodenmikrotiterplatten.

Vierfachansatz; 5 Tage Inkubation;

16 Std. $H^3$-Thymidineinbau.

Konzentrationen der Substanzen:

1 10 $\mu$g/ml

2 5 $\mu$g/ml

3 1 µg/ml
4 0.5 µg/ml
5 0.1 µg/ml
6 0.05 µg/ml
7 0.01 µg/ml

| µg/ml | α 2.3 × 10⁴ EL4-Zellen | | | | | | α 4.7 × 10⁴ EL4-Zellen | | | | | |
|---|---|---|---|---|---|---|---|---|---|---|---|---|
| | CsA (cpm) | % Hemmung | A/B (dto.) | dto. | C/D (dto.) | dto. | CsA (dto.) | dto. | A/B (dto.) | dto. | C/D (dto.) | dto. |
| 10 | 43 | -99,9 % | 672 | -99,0 % | 38 | -99,9 % | 31 | -99,9 % | 1024 | -98,4 % | 66 | -99,9 % |
| 5 | 89 | -99,9 % | 18108 | -74,3 % | 5507 | -92,2 % | 143 | -99,8 % | 47392 | -26,7 % | 5113 | -92,1 % |
| 1 | 848 | -98,8 % | 40113 | -43,2 % | 53037 | -25,9 % | 588 | -89,1 % | 46947 | -27,3 % | 40940 | -36,6 % |
| 0,5 | 1027 | -98,5 % | 58355 | -17,3 % | 61539 | -12,8 % | 1225 | -98,1 % | 60116 | -7,0 % | 42461 | -34,3 % |
| 0,1 | 2861 | -95,9 % | 53836 | -23,7 % | 63522 | -10,0 % | 2155 | -96,7 % | 62910 | -2,6 % | 52197 | -19,2 % |
| 0,05 | 3596 | -94,9 % | 56839 | -19,5 % | 50163 | -28,9 % | 4607 | -92,9 % | 69001 | +6,8 % | 48498 | -24,9 % |
| 0,01 | 22514 | -68,1 % | 57942 | -17,9 % | 39954 | -43,4 % | 24015 | -62,8 % | 64675 | +0,1 % | 50310 | -22,1 % |
| 0,00 | 70589 | | 70589 | | 70589 | | 64621 | | 64621 | | 64621 | |

SEQUENZPROTOKOLL

(1) ALGEMEINE INFORMATION:

    (i) ANMELDER:
        (A) NAME: HOECHST AKTIENGESELLSCHAFT
        (B) STRASSE: -
        (C) ORT: Frankfurt
        (D) BUNDESLAND: -
        (E) LAND: Deutschland
        (F) POSTLEITZAHL: 65926
        (G) TELEPHON: 069-305-5307
        (H) TELEFAX: 069-357175
        (I) TELEX: 41234-700 ho d

    (ii) ANMELDETITEL: Chrysospermine, Peptidwirkstoffe aus Apiocrea
        chrysosperma mit pharmakologischer Wirkung, ein Verfahren
        zur Herstellung und Verwendung derselben

   (iii) ANZAHL DER SEQUENZEN: 4

    (iv) COMPUTER-LESBARE FORM:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.25 (EPA)

(2) INFORMATION ZU SEQ ID NO: 1:

    (i) SEQUENZ CHARAKTERISTIKA:
        (A) LÄNGE: 19 Aminosäuren
        (B) ART: Aminosäure
        (D) TOPOLOGIE: linear

    (ii) ART DES MOLEKÜLS: Peptid

   (iii) HYPOTHETISCH: NEIN

   (iii) ANTISENSE: NEIN

    (vi) URSPRÜNLICHE HERKUNFT:
        (A) ORGANISMUS: Apiocrea chrysosperma
        (B) STAMM: DSM 7444

    (ix) MERKMALE:
        (A) NAME/SCHLÜSSEL: Peptide
        (B) LAGE: 1..19

    (ix) MERKMALE:
        (A) NAME/SCHLÜSSEL: Region
        (B) LAGE: 1
        (D) SONSTIGE ANGABEN: /label= misc_feature
            /note= "Xaa ist AcPhe (N-Acetylphenylalanin)"

    (ix) MERKMALE:
        (A) NAME/SCHLÜSSEL: Region
        (B) LAGE: 2
        (D) SONSTIGE ANGABEN: /label= misc_feature
            /note= "Xaa ist Abu"

    (ix) MERKMALE:
        (A) NAME/SCHLÜSSEL: Region
        (B) LAGE: 4
        (D) SONSTIGE ANGABEN: /label= misc_feature
            /note= "Xaa ist Abu"

```
(ix) MERKMALE:
     (A) NAME/SCHLÜSSEL: Region
     (B) LAGE: 5
     (D) SONSTIGE ANGABEN: /label= misc-feature
             /note= "Xaa ist Abu"

(ix) MERKMALE:
     (A) NAME/SCHLÜSSEL: Region
     (B) LAGE: 9
     (D) SONSTIGE ANGABEN: /label= misc_feature
             /note= "Xaa ist Abu"

(ix) MERKMALE:
     (A) NAME/SCHLÜSSEL: Region
     (B) LAGE: 10
     (D) SONSTIGE ANGABEN: /label= misc_feature
             /note= "Xaa ist Abu"

(ix) MERKMALE:
     (A) NAME/SCHLÜSSEL: Region
     (B) LAGE: 13
     (D) SONSTIGE ANGABEN: /label= misc_feature
             /note= "Xaa ist Abu"

(ix) MERKMALE:
     (A) NAME/SCHLÜSSEL: Region
     (B) LAGE: 15
     (D) SONSTIGE ANGABEN: /label= misc_feature
             /note= "Xaa ist Abu"

(ix) MERKMALE:
     (A) NAME/SCHLÜSSEL: Region
     (B) LAGE: 16
     (D) SONSTIGE ANGABEN: /label= misc_feature
             /note= "Xaa ist Abu"

(ix) MERKMALE:
     (A) NAME/SCHLÜSSEL: Region
     (B) LAGE: 17
     (D) SONSTIGE ANGABEN: /label= misc_feature
             /note= "Xaa ist Abu"

(ix) MERKMALE:
     (A) NAME/SCHLÜSSEL: Region
     (B) LAGE: 19
     (D) SONSTIGE ANGABEN: /label= misc_feature
             /note= "Xaa ist Trp-ol (Tryptophanol)"


(xi) SEQUENZBESCHREIBUNG: SEQ ID NO: 1:

Xaa Xaa Ser Xaa Xaa Leu Gln Gly Xaa Xaa Ala Ala Xaa Pro Xaa Xaa
1                5                    10                  15

Xaa Gln Xaa


(2) INFORMATION ZU SEQ ID NO: 2:

     (i) SEQUENZ CHARAKTERISTIKA:
         (A) LÄNGE: 19 Aminosäuren
         (B) ART: Aminosäure
         (D) TOPOLOGIE: linear

    (ii) ART DES MOLEKÜLS: Peptid

   (iii) HYPOTHETISCH: NEIN

   (iii) ANTISENSE: NEIN
```

28

```
(vi) URSPRÜNLICHE HERKUNFT:
     (A) ORGANISMUS: Apiocrea chrysosperma
     (B) STAMM: DSM 7444

(ix) MERKMALE:
     (A) NAME/SCHLÜSSEL: Peptide
     (B) LAGE: 1..19

(ix) MERKMALE:
     (A) NAME/SCHLÜSSEL: Region
     (B) LAGE: 1
     (D) SONSTIGE ANGABEN: /label= misc_feature
          /note= "Xaa ist AcPhe (N-Acetylphenylalanin)"

(ix) MERKMALE:
     (A) NAME/SCHLÜSSEL: Region
     (B) LAGE: 2
     (D) SONSTIGE ANGABEN: /label= misc_feature
          /note= "Xaa ist Abu"

(ix) MERKMALE:
     (A) NAME/SCHLÜSSEL: Region
     (B) LAGE: 4
     (D) SONSTIGE ANGABEN: /label= misc_feature
          /note= "Xaa ist Abu"

(ix) MERKMALE:
     (A) NAME/SCHLÜSSEL: Region
     (B) LAGE: 5
     (D) SONSTIGE ANGABEN: /label= misc_feature
          /note= "Xaa ist Abu"

(ix) MERKMALE:
     (A) NAME/SCHLÜSSEL: Region
     (B) LAGE: 9
     (D) SONSTIGE ANGABEN: /label= misc_feature
          /note= "Xaa ist Abu"

(ix) MERKMALE:
     (A) NAME/SCHLÜSSEL: Region
     (B) LAGE: 10
     (D) SONSTIGE ANGABEN: /label= misc_feature
          /note= "Xaa ist Abu"

(ix) MERKMALE:
     (A) NAME/SCHLÜSSEL: Region
     (B) LAGE: 13
     (D) SONSTIGE ANGABEN: /label= misc_feature
          /note= "Xaa ist Abu"

(ix) MERKMALE:
     (A) NAME/SCHLÜSSEL: Region
     (B) LAGE: 15
     (D) SONSTIGE ANGABEN: /label= misc_feature
          /note= "Xaa ist Iva (Isovalin)"

(ix) MERKMALE:
     (A) NAME/SCHLÜSSEL: Region
     (B) LAGE: 16
     (D) SONSTIGE ANGABEN: /label= misc_feature
          /note= "Xaa ist Abu"

(ix) MERKMALE:
     (A) NAME/SCHLÜSSEL: Region
     (B) LAGE: 17
     (D) SONSTIGE ANGABEN: /label= misc_feature
          /note= "Xaa ist Abu"
```

```
     (ix) MERKMALE:
          (A) NAME/SCHLÜSSEL: Region
          (B) LAGE: 19
          (D) SONSTIGE ANGABEN: /label= misc_feature
              /note= "Xaa ist Trp-ol (Tryptophanol)"


     (xi) SEQUENZBESCHREIBUNG: SEQ ID NO: 2:

     Xaa Xaa Ser Xaa Xaa Leu Gln Gly Xaa Xaa Ala Ala Xaa Pro Xaa Xaa
     1               5                   10                  15

     Xaa Gln Xaa


(2) INFORMATION ZU SEQ ID NO: 3:

     (i) SEQUENZ CHARAKTERISTIKA:
          (A) LÄNGE: 19 Aminosäuren
          (B) ART: Aminosäure
          (D) TOPOLOGIE: linear

     (ii) ART DES MOLEKÜLS: Peptid

     (iii) HYPOTHETISCH: NEIN

     (iii) ANTISENSE: NEIN

     (vi) URSPRÜNLICHE HERKUNFT:
          (A) ORGANISMUS: Apiocrea chrysosperma
          (B) STAMM: DSM 7444

     (ix) MERKMALE:
          (A) NAME/SCHLÜSSEL: Peptide
          (B) LAGE: 1..19

     (ix) MERKMALE:
          (A) NAME/SCHLÜSSEL: Region
          (B) LAGE: 1
          (D) SONSTIGE ANGABEN: /label= misc_feature
              /note= "Xaa ist AcPhe (N-Acetylphenylalanin)"

     (ix) MERKMALE:
          (A) NAME/SCHLÜSSEL: Region
          (B) LAGE: 2
          (D) SONSTIGE ANGABEN: /label= misc_feature
              /note= "Xaa ist Abu"

     (ix) MERKMALE:
          (A) NAME/SCHLÜSSEL: Region
          (B) LAGE: 4
          (D) SONSTIGE ANGABEN: /label= misc_feature
              /note= "Xaa ist Abu"

     (ix) MERKMALE:
          (A) NAME/SCHLÜSSEL: Region
          (B) LAGE: 5
          (D) SONSTIGE ANGABEN: /label= misc_feature
              /note= "Xaa ist Iva (Isovalin)"

     (ix) MERKMALE:
          (A) NAME/SCHLÜSSEL: Region
          (B) LAGE: 9
          (D) SONSTIGE ANGABEN: /label= misc_feature
              /note= "Xaa ist Abu"
```

```
(ix) MERKMALE:
     (A) NAME/SCHLÜSSEL: Region
     (B) LAGE: 10
     (D) SONSTIGE ANGABEN: /label= misc_feature
            /note= "Xaa ist Abu"

(ix) MERKMALE:
     (A) NAME/SCHLÜSSEL: Region
     (B) LAGE: 13
     (D) SONSTIGE ANGABEN: /label= misc_feature
            /note= "Xaa ist Abu"

(ix) MERKMALE:
     (A) NAME/SCHLÜSSEL: Region
     (B) LAGE: 15
     (D) SONSTIGE ANGABEN: /label= misc_feature
            /note= "Xaa ist Abu"

(ix) MERKMALE:
     (A) NAME/SCHLÜSSEL: Region
     (B) LAGE: 16
     (D) SONSTIGE ANGABEN: /label= misc_feature
            /note= "Xaa ist Abu"

(ix) MERKMALE:
     (A) NAME/SCHLÜSSEL: Region
     (B) LAGE: 17
     (D) SONSTIGE ANGABEN: /label= misc_feature
            /note= "Xaa ist Abu"

(ix) MERKMALE:
     (A) NAME/SCHLÜSSEL: Region
     (B) LAGE: 19
     (D) SONSTIGE ANGABEN: /label= misc_feature
            /note= "Xaa ist Trp-ol (Tryptophanol)"


(xi) SEQUENZBESCHREIBUNG: SEQ ID NO: 3:

Xaa Xaa Ser Xaa Xaa Leu Gln Gly Xaa Xaa Ala Ala Xaa Pro Xaa Xaa
1               5                   10              15

Xaa Gln Xaa


(2) INFORMATION ZU SEQ ID NO: 4:

     (i) SEQUENZ CHARAKTERISTIKA:
         (A) LÄNGE: 19 Aminosäuren
         (B) ART: Aminosäure
         (D) TOPOLOGIE: linear

     (ii) ART DES MOLEKÜLS: Peptid

     (iii) HYPOTHETISCH: NEIN

     (iii) ANTISENSE: NEIN

     (vi) URSPRÜNLICHE HERKUNFT:
          (A) ORGANISMUS: Apiocrea chrysosperma
          (B) STAMM: DSM 7444

     (ix) MERKMALE:
          (A) NAME/SCHLÜSSEL: Peptide
          (B) LAGE: 1..19
```

31

```
(ix) MERKMALE:
     (A) NAME/SCHLÜSSEL: Region
     (B) LAGE: 1
     (D) SONSTIGE ANGABEN: /label= misc_feature
         /note= "Xaa ist AcPhe (N-Acetylphenylalanin)"

(ix) MERKMALE:
     (A) NAME/SCHLÜSSEL: Region
     (B) LAGE: 2
     (D) SONSTIGE ANGABEN: /label= misc_feature
         /note= "Xaa ist Abu"

(ix) MERKMALE:
     (A) NAME/SCHLÜSSEL: Region
     (B) LAGE: 4
     (D) SONSTIGE ANGABEN: /label= misc_feature
         /note= "Xaa ist Abu"

(ix) MERKMALE:
     (A) NAME/SCHLÜSSEL: Region
     (B) LAGE: 5
     (D) SONSTIGE ANGABEN: /label= misc_feature
         /note= "Xaa ist Iva (Isovalin)"

(ix) MERKMALE:
     (A) NAME/SCHLÜSSEL: Region
     (B) LAGE: 9
     (D) SONSTIGE ANGABEN: /label= misc_feature
         /note= "Xaa ist Abu"

(ix) MERKMALE:
     (A) NAME/SCHLÜSSEL: Region
     (B) LAGE: 10
     (D) SONSTIGE ANGABEN: /label= misc_feature
         /note= "Xaa ist Abu"

(ix) MERKMALE:
     (A) NAME/SCHLÜSSEL: Region
     (B) LAGE: 13
     (D) SONSTIGE ANGABEN: /label= misc_feature
         /note= "Xaa ist Abu"

(ix) MERKMALE:
     (A) NAME/SCHLÜSSEL: Region
     (B) LAGE: 15
     (D) SONSTIGE ANGABEN: /label= misc_feature
         /note= "Xaa ist Iva (Isovalin)"

(ix) MERKMALE:
     (A) NAME/SCHLÜSSEL: Region
     (B) LAGE: 16
     (D) SONSTIGE ANGABEN: /label= misc_feature
         /note= "Xaa ist Abu"

(ix) MERKMALE:
     (A) NAME/SCHLÜSSEL: Region
     (B) LAGE: 17
     (D) SONSTIGE ANGABEN: /label= misc_feature
         /note= "Xaa ist Abu"

(ix) MERKMALE:
     (A) NAME/SCHLÜSSEL: Region
     (B) LAGE: 19
     (D) SONSTIGE ANGABEN: /label= misc_feature
         /note= "Xaa ist Trp-ol (Tryptophanol)"
```

```
(xi) SEQUENZBESCHREIBUNG: SEQ ID NO: 4:

Xaa Xaa Ser Xaa Xaa Leu Gln Gly Xaa Xaa Ala Ala Xaa Pro Xaa Xaa
1               5               10              15

Xaa Gln Xaa
```

**Patentansprüche**

1. Verbindung der allgemeinen Formel I


## AcPhe-Aib-Ser-Aib-x-Leu-Gln-Gly-Aib-Aib-Ala-Ala-Aib-Pro-y-Aib-Aib-Gln-Trp-ol


in der x und y unabhängig voneinander Aib oder Iva bedeuten.

2. Verbindung der allgemeinen Formel I zur Verwendung als Arzneimittel.

3. Arzneimittel, enthaltend Verbindung nach Anspruch 1 und einen oder mehrere pharmazeutische Träger.

4. Verfahren zur Herstellung einer Verbindung nach Anspruch 1, dadurch gekennzeichnet, daß man Apiocrea chrysosperma in einem Nährmedium kultiviert, bis sich eine Verbindung der allgemeinen Formel I in der Kulturbrühe anhäuft, und diese aus der Kulturbrühe isoliert.

5. Verfahren nach Anspruch 4, dadurch gekennzeichnet, daß man Apiocrea chrysosperma DSM 7444 kultiviert.

6. Verfahren nach einem oder mehreren der Ansprüche 4 - 5, dadurch gekennzeichnet, daß das Nährmedium 0.2 - 6 % Malzextrakt und 0.01 - 1 % Hefeextrakt enthält und man die Kultivierung bei einem pH-Wert zwischen 4 und 7 und einer Temperatur zwischen 15 - 35°C über 5 - 21 Tage durchführt.

7. Verfahren nach Anspruch 6, dadurch gekennzeichnet, daß das Nährmedium 2 - 4 % Malzextrakt und 0,1 - 0,4 % Hefeextrakt enthält und man die Kultivierung bei einem pH-Wert zwischen 5,5 und 6,5 und einer Temperatur zwischen 23 - 33°C über 7 - 18 Tage durchführt.

8. Verwendung einer Verbindung der allgemeinen Formeln I nach Anspruch 1 als pharmakologisch wirksamen Stoff.

9. Verwendung nach Anspruch 8 als Wirkstoff gegen Bakterien, Pilzen und/oder Nematoden.

10. Verwendung nach Anspruch 8 zur Herstellung von Arzneimitteln zur Behandlung von Abstoßungsreaktionen des Organempfängers gegen das verpflanzte Organ.

11. Verwendung nach Anspruch 10 zur Herstellung von Arzneimitteln, die während und/oder nach der Verpflanzung beim Empfänger oder Spender des Organs verabreicht werden.

12. Verwendung nach Anspruch 10 zur Herstellung von Arzneimitteln, die zur Behandlung von hyperakuten, akuten oder chronischen Abstoßungsreaktionen eingesetzt werden.

13. Verwendung nach Anspruch 10, dadurch gekennzeichnet, daß weitere Wirkstoffe wie Antiuricopathika, Thrombocytenaggregationshemmer, Analgetika und steroidale oder nichtsteroidale Antiphlogistika eingesetzt werden.

**14.** Verwendung nach Anspruch 8 zur Herstellung von Arzneimitteln zur Behandlung von Leukämie-, Sarkom-, Lymphknotentumor- oder Hautkrebserkrankungen.

**15.** Verwendung nach Anspruch 8 gegen Zellproliferation.

**16.** Apiocrea chrysosperma DSM 7444 , sowie dessen Varianten und Mutanten, soweit sie eine Verbindung der allgemeinen Formel I nach Anspruch 1 herstellen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,A | ANGEWANDTE CHEMIE INT. ED. ENGL. Bd. 18, Nr. 6 , 1979 Seiten 476 - 477 BRÜCKNER, H. ET AL. 'The sequences of the membrane-modifying peptide antibiotic Trychitoxin A-40' --- | | C07K7/08 A61K37/02 C12P21/02 //(C12P21/02, C12R1:645) |
| A | BIOMEDICAL MASS SPECTROMETRY Bd. 11, Nr. 11 , 1984 PRZYBILSKI, M. ET AL. 'Elucidation of the structure and microheterogenity of the polypeptide antibiotics Paracelsin and Trichotoxin A-50 ...' ----- | | |

| RECHERCHIERTE SACHGEBIETE (Int.Cl.5) |
|---|
| C07K A61K C12P C12R |

Der vorliegende Recherchenbericht wurde für alle Patentansprüche erstellt

| Recherchenort | Abschlußdatum der Recherche | Prüfer |
|---|---|---|
| MÜNCHEN | 27. April 1994 | Hermann, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...................................................................
& : Mitglied der gleichen Patentfamilie, übereinstimmendes Dokument

EPO FORM 1503 03.82 (P04C03)